# EUROPEAN PATENT SPECIFICATION

(11) **EP 3 645 070 B1**
(45) Date of publication and mention of the grant of the patent: **04.10.2023**
(21) Application number: 18737400.4
(22) Date of filing: 25.06.2018
(51) Int. Cl.: A61M 1/14, A61M 1/16, A61M 1/30

(54) **A DIALYSIS SYSTEM**
DIALYSESYSTEM
SYSTÈME DE DIALYSE

(30) Priority: 30.06.2017 GB 201710547
(43) Date of publication of application: 06.05.2020
(73) Proprietor: Quanta Dialysis Technologies Limited, Alcester Warwickshire B49 6EU (GB)
(72) Inventor: BUCKBERRY, Clive Henry, Alcester Warwickshire B49 6EU (GB)
(74) Representative: Range, Christopher William
(86) International application number: PCT/GB2018/051769
(87) International publication number: WO 2019/002837

(56) References cited:
- WO-A1-2015/022537
- US-A1- 2011 132 838
- US-A1- 2017 056 576
- US-B1- 6 303 036

## Description

### Field of invention

The invention relates to a dialysis system and method of operating the system.

### Background to the Invention

Patients suffering from kidney disorders rely on a variety of external blood treatments to remove the harmful waste substances that build up in their blood over time. One of the most common methods of treatment is haemodialysis.

Haemodialysis typically involves two networks of fluid passageways running adjacent to one another in a counter current flow arrangement. Blood is passed through one set of tubules and a cleaning solution is passed through the other. The pH and osmotic potential of the cleaning solution is adapted such that waste compounds built up in the blood diffuse from the blood into the cleaning solution via a semi permeable membrane which separates the blood and cleaning solution sides of the network of fluid passageways.

This provides a method of gradually removing waste materials from the blood, minimising fatigue to the patient. However, there are some disadvantages associated with haemodialysis not present with other forms of blood treatment.

Many mid-size and large-size waste solutes dissolved in the blood (including such as proteins and polypeptides) are difficult to remove completely from the blood using diffusion alone and it can take a long time to reduce the levels of these substances in the blood to acceptable levels. An alternative approach is to use haemodiafiltration.

Haemodiafiltration involves administering sterile cleaning solution to the blood either by employing a large hydrostatic potential to force sterile cleaning solution across a semi permeable membrane into the blood or by directly adding it to the blood; and then pulling the sterile cleaning solution, complete with dissolved waste products, back across the semi permeable membrane for subsequent disposal.

Examples of haemodiafiltration machines are disclosed in, for example "Lee, K., et al., Evaluation of a New Method for Pulse Push/Pull Hemodialysis: Comparison with Conventional Hemodialysis, ASAIO Journal, 2012, page 232 - 237".

This type of blood treatment is not limited by diffusion as sterile cleaning solution is allowed to mix directly with the blood. However, the rapid extraction of waste products from a patient's blood regularly leaves patients fatigued.

In both methods, it is conventional to take blood from a patient's artery via a cannula into an arterial line, then a pump and into the dialyser cartridge. Treated blood then leaves the cartridge via a venous line and passes back to the patient's vein by a venous cannula.

It is also known to use a single venous access for removal from, and return to, the body, of blood for treatment. When using a single venous access removal and return of blood must be done sequentially.

Single venous access may mitigate the possibility of exsanguination due to undetected venous needle dislodgement and is therefore seen as a desirable means of dialysing nocturnally.

Three methods of single needle dialysis are known in the prior art. The first uses two peristaltic pump that exchange a volume of blood between them. The second utilizes a single peristaltic pump displacing blood to an accumulator located in the extracorporeal circuit. In a single peristaltic pump system the accumulator is used to store the removed blood while the system is switched over to allow blood to be returned to the patient. Those methods are described by Polaschegg, in Haemodialysis Machines and Monitors, Replacement of Renal Function by Dialysis Handbook, page 365 to 368, Revised 5th Edition. Both methods are known to be more complex and require different tube sets from those used with dual needle access. In addition, the exchange of molecules across the dialyser is driven by diffusion.

The third method is to use a single, dual lumen needle but this is subject to potential high recirculation between the two lumens of the needle.

It is an object of the invention to provide an improved dialysis system. US 2017/056576 discloses a blood plasma and red blood cell generation device. US 6303036 discloses a method and apparatus for haemodiafiltration. WO 2015/022537 discloses a blood treatment device which can perform both haemodialysis and haemodiafiltration.

### Summary of the Invention

In a first aspect of the invention, there is provided a dialysis system comprising a dialysate fluid circuit and a blood circuit and a dialyser, the dialysate fluid circuit comprising a dialysate input pump having a dialysate input pump chamber, and a dialysate output pump having a dialysate output pump chamber,
the blood circuit comprising a blood pump, a blood plasma accumulator chamber a blood removal line (14a) upstream of the dialyser (12) a blood return line (14b) downstream of the dialyser (12) and a venous clamp (124) arranged on the blood return line (14b),
the dialyser comprising a fluid chamber with a blood filter arranged in the fluid chamber to divide the system into a blood side and a dialysate side, the dialyser having a dialysate inlet and a dialysate outlet and a blood inlet and a blood outlet,
the dialysate fluid circuit being arranged on the dialysate side of the system,
the blood accumulator chamber being arranged on the dialysate side of the system, wherein the system includes a control processor configured to close the venous clamp to pressurize blood in the blood circuit so that blood plasma is forced onto the dialysate side of the system.

By arranging the blood accumulator chamber on the dialysate side of the system, a blood accumulator is provided to allow a single venous access, whilst passing blood across the dialyser filter to reach the blood accumulator mimics the action of haemodiafiltration described above (only in reverse).

Most preferably, the dialysate output pump chamber comprises the blood accumulator chamber. This allows the dialysate output pump to act to return the blood to the patient.

The dialysate input pump chamber may have a dialysate inlet, with a dialysate inlet valve, in fluid communication with a source of dialysate and a dialysate outlet, with a dialysate outlet valve, in fluid communication with the dialysate inlet of the dialyser.

The dialysate output pump chamber may have a spent dialysate inlet, with a spent dialysate inlet valve, in fluid communication with the dialysate outlet of the dialyser and a spent dialysate outlet, with a spent dialysate outlet valve, in fluid communication with a drain.

The dialysate fluid circuit preferably comprises a first pump having a first pump chamber with a fresh dialysate inlet, with a fresh dialysate inlet valve, in fluid communication with a source of dialysate, a dialyser fluid line, with a dialyser fluid valve, in fluid communication with dialyser and a drain line with a drain valve, in fluid communication with a drain; and a second pump, having a second pump chamber with a fresh dialysate inlet, with a fresh dialysate inlet valve, in fluid communication with a source of dialysate, a dialyser fluid line, with a dialyser fluid valve, in fluid communication with a dialyser and a drain line with a drain valve in fluid communication with a drain; a control processor which controls the valves whereby the first pump can be configured to operate as the dialysate input pump by closing off the drain valve and the second pump is then configured to operate as the dialysate output pump by closing off the respective fresh dialysate inlet valve and vice versa. In such a case, the pump chamber of either pump can be configured to operate as the blood accumulator chamber by closing off the respective drain valve and the respective fresh dialysate inlet valve thereof.

The dialysate fluid circuit preferably comprises a body defining the pump chambers closed by a membrane, which is actuable to effect pumping. The body and membrane may form a cartridge for use with a machine which includes actuators to actuate the membrane to effect pumping. The valves are preferably defined on the body and the valves are opened and closed by actuation of the membrane. The cartridge may also include at least one mixing chamber to effect mixing of dialysate constituents into a mixed dialysate and the mixing chamber is in fluid communication with the dialysate input pump.

According to a second aspect of the invention, there is provided a method of testing a dialysis system comprising the steps of a) providing a dialysis system according to the first aspect of the invention, b) providing a source of dialysate fluid, c) providing a source of blood analogue fluid, d) supplying the dialysate fluid to the dialysate circuit and supplying the blood analogue fluid to the blood circuit, e) operating the dialysate output pump to draw blood analogue fluid, and/or operating the blood pump to push blood analogue fluid, across the blood filter onto the dialysate side and into the dialysate output pump chamber, f) deactivating the blood pump and operating the dialysate output pump to push blood analogue fluid back across the blood filter onto the blood side of the system.

The blood analogue fluid is a fluid designed to behave in a similar fashion to blood to enable the device to be tested. Blood plasma may be used as the analogue fluid. The blood analogue fluid preferably includes a marker, such as a dye or a marker molecule which may be sensed using appropriate sensors.

Preferably step a) further comprises providing the blood circuit of the dialysis system with a blood inlet line upstream of the dialyser, a blood return line downstream of the dialyser and a blood return line closure arranged on the blood return line or at the blood outlet of the dialyser, and step e) comprises a first pre-step e' in which the blood pump is deactivated, the blood return line closure is closed to prevent flow of blood analogue fluid from the dialyser to the blood return line, the dialysate input pump chamber is filled with dialysate, the dialysate outlet valve of the dialysate input pump chamber is open, the dialysate output pump chamber is empty, the spent dialysate inlet valve is open and the spent dialysate outlet valve is closed, the first pre-step e' comprising actuating the dialysate input pump to push fresh dialysate from the dialysate input pump chamber to the dialyser and simultaneously actuating the dialysate output pump to draw spent dialysate from the dialyser into the dialysate output pump chamber; and a second pre-step e", in which the blood pump is deactivated, blood return line closure is closed, the dialysate input pump chamber is empty and the dialysate output pump chamber is filled with spent dialysate from first pre-step e', second pre-step e" comprising opening the dialysate inlet valve and closing dialysate outlet valve of the dialysate input pump, closing the spent dialysate inlet valve of the dialysate output pump, opening spent dialysate outlet valve, then actuating dialysate input pump to cause fresh dialysate to flow from the dialysate source into the dialysate input pump chamber and simultaneously actuating the dialysate output pump to push spent dialysate to drain.

Preferably, the dialysate input and output pumps are membrane pumps. The membrane pumps typically comprise a chamber which is adapted to hold a volume of solution and a membrane sealing the chamber. The membrane can be forced down into the chamber to expel the solution from the chamber. The membrane is often a flexible membrane and is typically fabricated from an elastic material. The elastic material is often made from a plastic or polymeric material and typically forms a film sealing one end of the chamber. The membrane may extend substantially over all the chambers used in the device or each membrane pump may comprise a separate membrane in communication with the chambers.

Preferably, the pumps are arranged to pump a predetermined volume of dialysate. Typically, the pumps used in the invention are adapted to pump the same volume of dialysate as each other. Where the pumps are positive displacement pumps, the pumps are adapted to pump the same volume of solution as each other in each single stroke. This ensures that the amount of solution pumped into the dialyser by the first pump is the same as the amount of solution drawn from the dialyser by the second pump.

Typically, the dialysate input pump and dialysate output pump are both operable either to deliver a volume of dialysate from a dialysate source to the dialyser or remove a volume of dialysate from the dialyser and deliver said dialysate to a drain. Adapting both pumps to function in this way allows the roles of each pump to be periodically swapped. This is usually done at regular intervals in order to negate any small manufacturing discrepancies in the volume of the pump chambers.

The dialysate pumps may be formed on a disposable cartridge. Typically the dialysate source, pumps, valves and fluid passageways are all contained on the cartridge.

### Brief description of the drawings

The invention will now be described with reference to the following figures.
Figure 1 shows a schematic of a dialysis system having a disposable cartridge comprising a fluid path defined by pumps and valves.
Figure 1a shows a detailed schematic view of the cartridge of Figure 1.
Figure 2 shows a schematic view of the operation of a pump of the type defined by the disposable cartridge.
Figure 3 shows a schematic view of the pump and valve arrangement of the invention.
Figures 4a to 4e are schematic views of a dialysis system in accordance with the first aspect of the invention, performing the method of the second aspect of the invention.

### Description

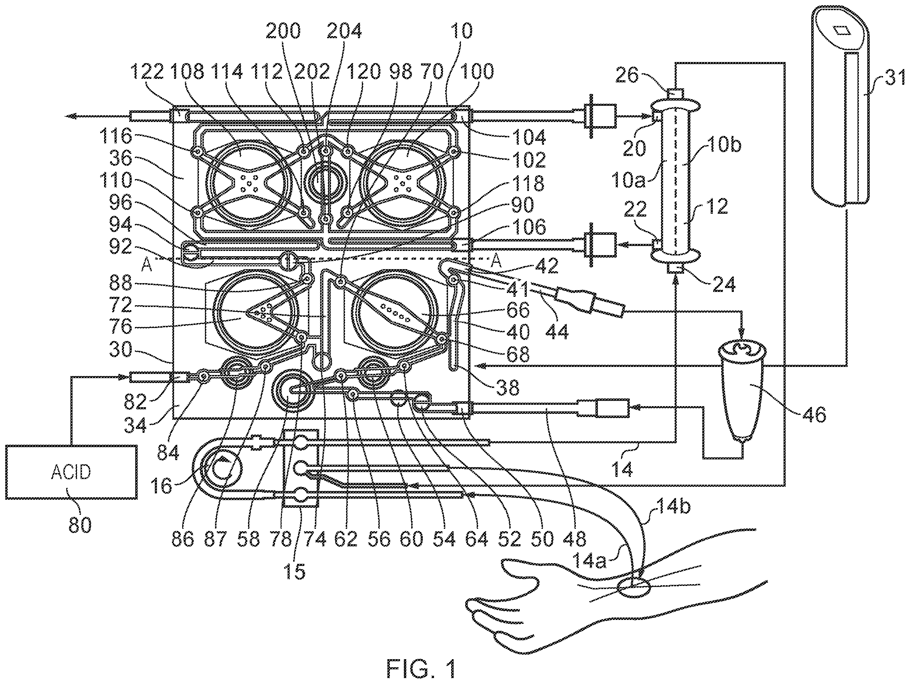

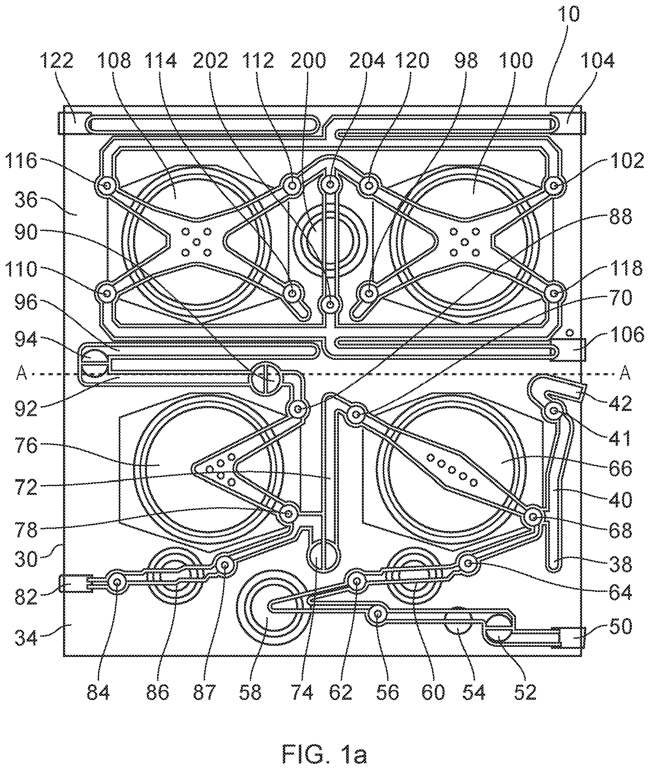

Referring to Figures 1 and 1a, a dialysis system, generally referred to as 10, is shown. A dialyser 12 receives blood via a blood removal venous line 14a connected to a patient by a vascular access device (not shown for clarity), for example a hollow needle as typically used for drawing blood from a patient. The blood is pumped from the patient via a blood pressure sensor module 15 to the dialyser by a peristaltic pump 16. The blood passes through the dialyser 12 in a known manner and is returned to the patient via a return venous line 14b connected to the same vascular access device as the blood removal venous line 14a. The dialyser 12 comprises a cylindrical tube closed by opposing ends. A semi-permeable membrane (not shown) is provided within the dialyser tube and separates the patient's blood from a dialysate (cleaning) solution. The membrane extends substantially between the opposing ends of the cylinder. The dialysate solution removes impurities from the patient's blood in a known manner.

The dialyser has an inlet 20 for receiving clean dialysate solution and an outlet 22 for removing spent dialysate solution from the dialyser 12. The dialyser also has an inlet 24 for receiving untreated blood from the peristaltic pump 16 and an outlet 26 for returning processed blood to the patient. The dialyser 12 is typically provided in a substantially upright orientation, in use, with the patient's blood flowing longitudinally through the dialyser 12 from the blood inlet 24 to the blood outlet 26. The dialysate solution inlet 20 and dialysate solution outlet 22 are configured to be orientated substantially orthogonal to the blood inlet 24 and blood outlet 26, and to provide a counter-flow. Dialysate solution is circulated through the haemodialysis machine at a fluid flow rate in the region of 400ml/min for approximately four hours.

The semi-permeable membrane in the dialyser 12 divides the system 10 into a dialysate side 10a and a blood side 10b.

The dialysis system 10 defines a fluid circuit including a cartridge 30, as will now be described. The cartridge 30 is a consumable component in the haemodialysis machine described.

The cartridge 30 is formed from an acrylic plastic such as SG-10 and has a machine side and a patient side. The cartridge 30 defines pump chambers which are closed by respective diaphragms, formed from, for example, DEHP-free PVC, to define respective pumps. In this embodiment, each diaphragm is part of a single, common sheet of material applied to the cartridge 30. The individual diaphragms are operable by pneumatic pressure applied thereto.

A series of flow paths are formed in the cartridge 30 for carrying dialysate solution constituted from water, bicarbonate solution and acid solution. The flow paths are located between the sheet of material closing the machine side of the cartridge 30 and a further sheet of the same material closing the patient side of the cartridge 30.

The cartridge 30 is inserted into a machine which is provided with a series of pneumatic pumps and valves and a corresponding, opposing layout of pump chambers to match the cartridge pump chambers.

In use, a pressure source applies either a positive or negative pressure to one side of the diaphragm of each pump chamber, as required, pulling and pushing the diaphragm back and forth to pump fluid through the fluid paths in the cartridge 30, in a circuit defined by a plurality of valves, which also work by movement of the membrane back and forth

The valves of the cartridge 30 are conventional diaphragm valves defined by respective openings in the cartridge 30 and closed by respective flexible diaphragms. Each valve is operable by applying a negative pressure to the diaphragm to open the valve and applying a positive pressure to the diaphragm to close the valve. The diaphragm of each valve is part of the single, common sheet of material applied to the machine side of the cartridge 30. The valves are opened and closed according to a flow control strategy, as will become apparent.

The machine side of the cartridge 30 abuts a pump driver (not shown) comprising a platen having a plurality of recessed surfaces, each recessed surface substantially corresponding in geometry and volume to a pump chamber defined in the cartridge 30. Each recessed surface has a fluid port connectable with a source of positive fluid, pressure and, with a source of negative fluid pressure via a valve.

The positive and negative fluid pressure sources, typically pneumatic pressure, include a pressure pump and a vacuum pump respectively. When the valve is operated to allow fluid to flow into a recessed surface from the source of positive fluid pressure, the diaphragm moves into a corresponding pump chamber and any fluid, i.e. dialysate solution, therein is expelled from that pump chamber via the series of flow paths. When the valve is operated to allow fluid to flow out of a recessed surface to the source of negative fluid pressure, the diaphragm is moved away from a pump chamber and into the corresponding recessed surface to permit fluid to be drawn into that pump chamber via the series of flow paths. The surface of the pump chambers and of the platen provide a positive stop for each diaphragm, to prevent overstretching thereof. The positive stop ensures that the volume of fluid drawn into and pumped from the pump chambers is accurately controlled.

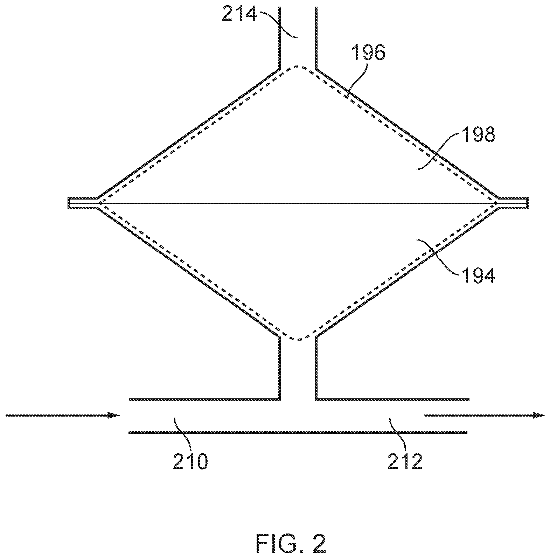

The cartridge 30 has two main functions, preparation of dialysate solution and flow balance. Each function is performed by a separate part of the cartridge as illustrated in Figures 1 and 2 by the schematic separation of the cartridge into two parts by the line A-A in the figures. The dialysate preparation function is performed by one part of the cartridge, generally referred to at 34 and the flow balance function is performed by the other part of the cartridge, generally referred to at 36. The cartridge 30 prepares an accurately mixed homogenous dialysate solution and ensures that the flow of clean dialysate supplied to the dialyser 12 matches (to within clinical tolerances) the volume of spent dialysate drawn from the dialyser 12.

The cartridge 30 is provided with a plurality of connections to and from the cartridge 30 as described below.

A first inlet port 38, from hereon referred to as the water inlet port, defined in the machine side of the cartridge 30 receives purified water from a purified water supply 31 such as a reverse osmosis water supply.

A first outlet port 42, from hereon referred to as the water outlet port, defined in an edge of the cartridge 30 directs the purified water to a first dialysate solution constituent which, in the illustrated embodiment shown in Figures 1 and 1a, is bicarbonate 46.

A second inlet port 50, from hereon referred to as the bicarbonate inlet port, defined in the same edge of the cartridge 30 as the water outlet port 42 receives purified water mixed with the bicarbonate 46.

A third inlet port 82, from hereon referred to as the acid inlet port, defined in the opposite edge of the cartridge 30 to the water outlet port 42 and bicarbonate inlet port 50 receives a second dialysate solution constituent which, in the illustrated embodiment shown in Figures 1 and 1a, is acid 80.

A second outlet port 104, from hereon referred to as the clean dialysate solution outlet port, is defined in the same edge of the cartridge as the water outlet port 42 and the bicarbonate inlet port 50. The clean dialysate outlet port 104 directs clean dialysate solution to the dialyser 12.

A fourth inlet port 106, from hereon referred to as the spent dialysate solution inlet port, is defined in the same edge of the cartridge 30 as the water outlet port 42, bicarbonate inlet port 50 and clean dialysate outlet port 104. The spent dialysate solution inlet port 106 receives spent dialysate solution from the dialyser 12.

A third outlet port 122, from hereon referred to as the drain port, is defined in the same edge of the cartridge as the acid inlet port 82. The drain port 122 directs spent dialysate solution out of the cartridge 30.

### Dialysate Preparation

Dialysate solution is prepared in the cartridge 30 by combining purified water with two dialysate constituents, namely a bicarbonate solution and an acid solution.

Purified water is admitted into the cartridge 30 from a purified water supply 31 via the water inlet port 38. The purified water passes through a channel 40 via a water inlet valve 41, when open, and exits the cartridge 30 at the water outlet port 42. From here, the purified water is carried by a tube 44 through a bicarbonate cartridge 46 in a known manner to generate a purified water and bicarbonate solution. The purified water and bicarbonate solution is carried by a tube 48 and re-admitted into the cartridge 30 via the bicarbonate inlet port 50.

The temperature of the bicarbonate solution is measured at sensing port 52 and the bicarbonate solution pressure is measured at sensing port 54. The bicarbonate solution passes a bicarbonate control valve 56, when open, before entering a bicarbonate solution reservoir 58 having an inlet and an outlet. The bicarbonate control valve 56 is closed when flow therethrough is not required.

A bicarbonate dosing pump chamber 60 has an inlet and an outlet and receives the bicarbonate solution from the bicarbonate solution reservoir 58 through a bicarbonate dosing pump inlet valve 62. The bicarbonate dosing pump chamber 60 is closed by a diaphragm to define a bicarbonate dosing pump which, upon actuation of the diaphragm, pumps the bicarbonate solution from the bicarbonate dosing pump 60 to a first mixing pump chamber 66 (bicarbonate pump chamber). The bicarbonate dosing pump 60 has a bicarbonate dosing pump outlet valve 64 which is closed when the bicarbonate dosing pump inlet valve 62 is open. The bicarbonate dosing pump outlet valve 64 is opened to permit bicarbonate solution to be pumped to the bicarbonate pump chamber 66. When the bicarbonate dosing pump outlet valve 64 is open, the bicarbonate dosing pump inlet valve 62 is closed to prevent bicarbonate solution from being pumped back into the bicarbonate solution reservoir 58.

The bicarbonate pump chamber 66 having an inlet and an outlet receives the purified water and bicarbonate solution from the bicarbonate dosing pump 60 via a bicarbonate pump inlet valve 68. The bicarbonate pump inlet valve 68, when open, can also admit purified water into the bicarbonate pump chamber 66 from the water inlet port 38. The bicarbonate pump chamber 66 is closed by a diaphragm to define a pump which, upon actuation of the diaphragm, pumps the bicarbonate solution and purified water therein through a bicarbonate pump outlet valve 70 to a second mixing pump chamber 76 (acid pump).

When the bicarbonate pump inlet valve 68 is open, the bicarbonate pump outlet valve 70 and water outlet valve 41 are closed. When the bicarbonate pump outlet valve 70 is open, the bicarbonate pump inlet valve 68 is closed to prevent the bicarbonate and purified water solution from being pumped back into channel 40.

From the bicarbonate pump outlet valve 70, the bicarbonate and purified water solution enters a sensor channel 72 in which the haemodialysis machine measures the conductivity of the bicarbonate and purified water solution in a known manner. The bicarbonate and purified water solution then enters a temperature sensor 74 before, if the conductivity and temperature of the bicarbonate and purified water solution are within tolerance, entering the acid pump chamber 76.

The acid pump chamber 76 having an inlet and an outlet receives the bicarbonate and purified water solution from the bicarbonate pump 66 via an acid pump inlet valve 78. The acid pump inlet valve 78, when open, can also admit an acid solution into the pump chamber 76. The acid pump chamber 76 is closed by a diaphragm to define a pump which, upon actuation of the diaphragm, pumps the acid solution, bicarbonate solution and purified water therein through an acid pump outlet valve 88 to the first flow balance pump chamber 100. When the acid pump inlet valve 78 is open, the acid pump outlet valve 88 is closed. When the acid pump outlet valve 88 is open, the acid pump inlet valve 78 is closed.

The acid solution is admitted into the cartridge 30 from a pre-determined supply of acid 80 via the acid solution inlet port 82. From the acid solution inlet port the acid solution passes through an acid dosing pump chamber 86 via an acid dosing pump inlet valve 84 and an acid dosing pump outlet valve 87. The acid dosing pump outlet valve 87 is closed when the acid dosing pump inlet valve 84 is open. The acid dosing pump inlet valve 84 is closed when the acid dosing pump outlet valve 87 is open.

The dialysate solution exits the acid pump chamber via the acid pump outlet valve 88 and passes through a first dialysate solution temperature sensor 90 and a first dialysate solution conductivity sensor 92. A second dialysate solution temperature sensor 94 and a second dialysate solution conductivity sensor 96 are provided to corroborate the data provided by the first dialysate solution temperature sensor 90 and the first dialysate solution conductivity sensor 92. Providing the data measured by sensors 90, 92, 94 and 96 is within tolerance, the dialysate solution is admitted into a first flow balance pump chamber 100

### Flow Balance

The flow balance function of the cartridge 30 provides first and second flow balance pump chambers 100, 108, each having two inlets and two outlets to define two independent flow paths therethrough. The first and second flow balance pump chambers 100, 108 are of substantially equal volume. Either the first or second flow balance pump chamber 100, 108 pumps dialysate solution to a dialyser 12 and the other of the first or second flow balance pump chambers 100, 108 pumps dialysate solution from the dialyser 12 to the drain port 122. After every approximately 20 strokes of the first and second flow balance pumps 100, 108, their function is reversed.

From this point onwards, dialysate solution will be referred to as either clean dialysate solution or spent dialysate solution. Clean dialysate solution is intended to mean dialysate solution that is either new dialysate solution or clean dialysate solution that has been treated to remove waste product therefrom. Spent dialysate solution is intended to mean dialysate solution that has passed through the dialyser 12 to remove waste fluids from a patient's blood into the dialysate solution.

Each of the first and second flow balance pump chambers 100, 108 are closed by a diaphragm to define respective pumps. The diaphragm is actuated away from a pump chamber by a negative pressure source to draw a volumetrically measured quantity of dialysate solution into the pump chamber. The diaphragm is actuated toward the pump chamber to pump the fluid therein out of an outlet.

The first flow balance pump chamber 100 has a clean dialysate solution inlet valve 98 for receiving clean dialysate solution from the acid pump 76 and a clean dialysate solution outlet valve 102 for pumping clean dialysate solution to the dialyser 12. The first flow balance pump chamber 100 also has a spent dialysate solution inlet valve 118 for receiving spent dialysate from the dialyser 12 and a spent dialysate solution outlet valve 120 for pumping the spent dialysate to drain via drain outlet port 122.

At any one time, only one of valves 98, 102, 118 or 120 will be open and the other three valves will be closed. The flow balance function, as described above, requires alternating the function of each flow balance pump approximately every 20 cycles. Therefore, when the first flow balance pump 100 is pumping clean dialysate solution to the dialyser 12, only valves 98 and 102 are in use and when the first flow balance pump 100 is pumping spent dialysate solution from the dialyser 12 to drain, only valves 118 and 120 will be in use.

The clean dialysate solution is pumped out of the first flow balance pump chamber 100 through the first flow balance pump clean dialysate solution outlet valve 102, upon closure of the first flow balance pump clean dialysate inlet valve 98, to the dialyser 12 via the dialyser outlet port 104.

Spent dialysate solution returns to the cartridge 30 from the dialyser 12 via the dialyser inlet port 106. The second flow balance pump chamber 108 has a spent dialysate solution inlet valve 110 for receiving spent dialysate solution from the dialyser 12 and a spent dialysate solution outlet valve 112 for pumping the spent dialysate solution to drain via drain outlet port 122. The second flow balance pump 108 also has a clean dialysate solution inlet valve 114 for receiving clean dialysate solution from the acid pump chamber 76 and a clean dialysate solution outlet valve 116 for pumping clean dialysate solution to the dialyser 12.

At any one time, only one of valves 110, 112, 114, 116 will be open and the other three valves will be closed. When the second flow balance pump 108 is pumping clean dialysate solution to the dialyser 12, only valves 114 and 116 will be in use and when the second flow balance pump 108 is pumping spent dialysate solution from the dialyser 12 to drain, only valves 114 and 116 will be in use.

In the illustrated example, the operation of the first and second flow balance pumps 100, 108 can be switched so that the first flow balance pump 100 is used to draw spent dialysate solution from the dialyser 12 and the second flow balance pump 108 is used to pump clean dialysate solution into the dialyser 12 as described below.

The clean dialysate solution is drawn into the second flow balance pump chamber 108 from the acid pump 76 via the second flow balance pump clean dialysate solution inlet valve 114 upon actuation of the diaphragm. The clean dialysate solution is then pumped from the second flow balance pump chamber 108 via the second flow balance pump clean dialysate solution outlet valve 116, upon closure of the clean dialysate solution inlet valve 114, to the dialyser 12.

Spent dialysate solution from the dialyser 12 is drawn into the first flow balance pump 100 via the second flow balance pump spent dialysate solution inlet valve 118. The spent dialysate solution is then pumped out of the first flow balance pump chamber 100 via the second flow balance pump spent dialysate solution outlet valve 120, upon closure of the spent dialysate solution inlet valve 118, to drain via drain outlet port 122.

The volume of fluid that is returned from the dialyser 12 is greater than the volume of fluid that is pumped to the dialyser via the first or second flow balance pump 100, 108. The first and second flow balance pumps have fixed volumes meaning that the excess fluid volume cannot be accommodated in the first or second flow balance pump.

An ultrafiltration pump 200 is provided between the first and second flow balance pumps 100, 108 and has an inlet valve 210 and an outlet valve 212. The ultrafiltration pump 200 comprises a concave recess in the cartridge closed by a flexible diaphragm, the concave recess and the flexible diaphragm defining an ultrafiltration pump chamber.

In use, the inlet valve 210 of the ultrafiltration pump 200 is opened to allow the ultrafiltration pump to draw in a pre-determined volume of spent dialysate solution. When the inlet valve 210 of the ultrafiltration pump is open, the outlet valve 212 of the ultrafiltration pump 200 is closed. When the ultrafiltration pump 200 has received a volume of spent dialysate solution, the outlet valve 212 is opened and the spent dialysate solution in the ultrafiltration pump chamber is pumped through the outlet valve 212 to drain via the drain outlet port 122. When the outlet valve 212 of the ultrafiltration pump 200 is open, the inlet valve 210 of the ultrafiltration pump 200 is closed.

The purpose of the ultrafiltration pump is to remove excess fluid generated by the patient. By separating the ultrafiltration pump operation from the flow balance pumps and by employing a pump arrangement described herein, fluid can be removed from the dialyser at appropriate intervals between the stages of the operation of the flow balance pumps, without requiring modification to the flow balance pump operation. Usually, the ultrafiltration pump will remove fluid from the dialyser during a pump swapping operation of the flow balance pumps and this may be done in the range of once every 10 to once every 30 flow balance pump cycles. Typically, fluid is removed from the dialyser by the ultrafiltration pump approximately once every 20 pump cycles.

Figure 2 shows a representative view of a flow balance pump 100 according to the present invention. The flow balance pump chamber 194 is provided on the cartridge and is closed by a diaphragm 196 which, at rest, sits across the pump chamber 194. The pump chamber receives either clean or spent dialysate solution via a dialysate solution inlet port 210 and pumps dialysate solution from the pump chamber via a dialysate solution outlet port 212.

The cartridge 30 is removably mounted into a haemodialysis machine which has a flow balance pump cavity 198 substantially corresponding in dimension and shape to the pump chamber 194. Upon supply of positive or negative pressure via a pump cavity pressure inlet port 214, the diaphragm is actuated into either the pump chamber 194 or pump cavity 198 to either draw fluid into the pump chamber 194 or pump fluid from the pump chamber 194.

### Operation of the Device

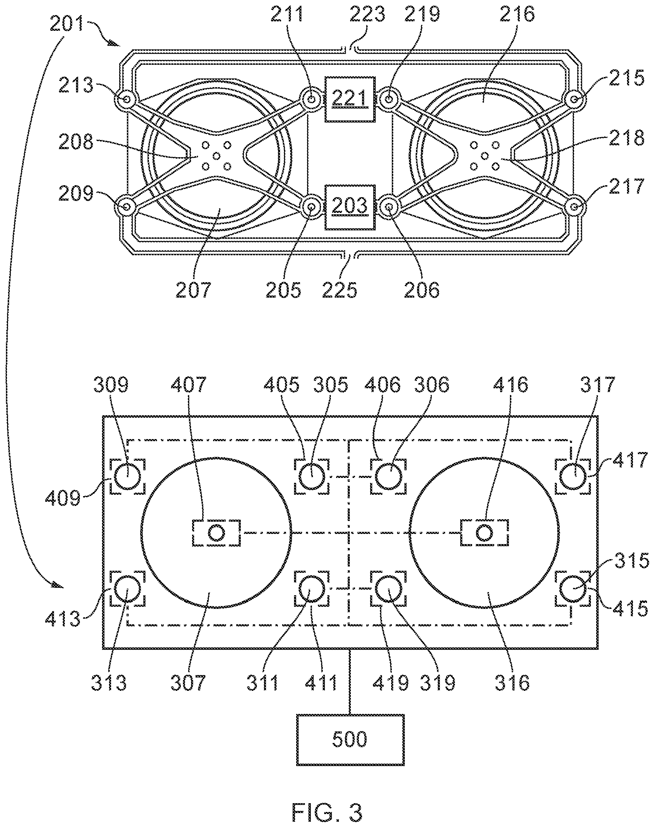

Figure 3 shows a schematic representation of the pump and valve arrangement 201 of the invention. In this case, the pump and valve arrangement 201 is provided by the combination of a membrane pump cartridge (or part cartridge) and a vacuum pump array with platen. The membrane pump cartridge is similar in layout to the flow balance pump arrangement described above.

The membrane pump cartridge comprises first and second source valves 205, 206, first and second pumps 207, 216 and first and second pump chambers 208, 218, first and second dialyser inlet valves 209, 217 and first and second dialyser outlet valves 213, 215.

The vacuum pump array and platen comprises a platen having a pattern of circular depressions which correspond in position and size to the valves and pumps on the pump cartridge. In the figure, these are numbered 100 higher than the membrane pump features.

Each depression has an aperture at the base thereof which is in fluid communication with an associated vacuum pump. Each vacuum pump, shown in broken lines as they sit on the rear face of the platen, is numbered 100 higher than the respective associated platen feature.

All of the vacuum pumps are connected to a control system 500. The control system 500 is a microprocessor which operates the vacuum pumps 405-419 in a manner so as to effect either haemodiafiltration or haemodialysis. The connection to the pumps may be wired or wireless. Wireless connection options include IR, Bluetooth or WIFI, amongst others.

The dialysate is produced elsewhere on the cartridge by mixing acid and bicarbonate compounds with a set volume of de-ionised, water provided by a reverse osmosis machine which has been sterilised as is described above. This forms the source of dialysate 327 used by the pump and valve arrangement 201.

By selectively operating the vacuum pumps, the control system controls the opening and closure of the valves as well as actuation of the first and second pumps. The microprocessor control system is programmable to operate the valves in a variety of different configurations. Based on the programming of the controller, the controller will communicate with each of the valves or means for operating the valves, so that each valve may be opened and closed independently based on the programming entered into the controller by the user, skilled operator or programme instructions.

Although the control system 500 has been described in the specific embodiment as a microprocessor, the control system 500 may instead comprise an electrical switching arrangement or a mechanical control arrangement. In the case of a mechanical control arrangement, rather than individual vacuum pumps for each platen cavity, it is envisaged that a single vacuum pump would apply a negative pressure to the platen and a mechanical camming or gearing arrangement would actuate valves on the platen to control the application of the negative/positive pressure selectively according to the required operating mode.

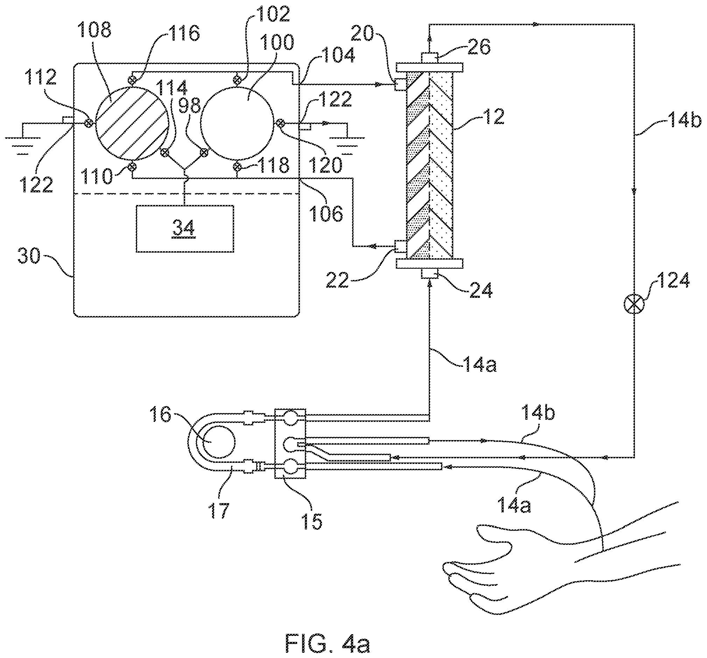

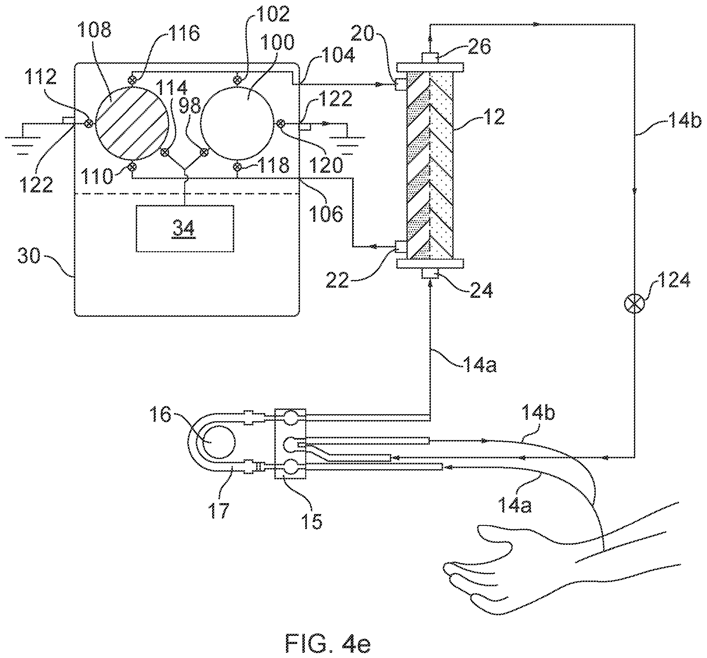

Referring to Figures 4a to 4e, a dialysis system 10 in accordance with the first aspect of the invention operating a method in accordance with the second aspect of the invention is shown schematically. Figures 4a to 4e show a simplified view of the system shown in Figure 1 with the water supply, bicarbonate cartridge and acid supply omitted for clarity. Also, the representation of the mixing part of the cartridge 34 of the cartridge 30 has been simplified but corresponds to the arrangement shown in Figures 1 and 2. The system 10 comprises a blood circuit and a dialysate circuit. The blood circuit comprises the blood removal line 14a, the blood pressure sensor modular 15, a tube set 17 of the peristaltic pump 16, the dialyser 12, a blood accumulator chamber and the blood return line 14b, which has a blood return line closure thereon in the form of a venous clamp 124. The dialysate fluid circuit comprises the first and second flow balance pumps, the clean dialysate solution inlet line and the spent dialysate solution outlet line and the drain outlet line.

Figure 4a illustrates the system in an initial condition. In this condition, the peristaltic pump 16 is inactive and the venous clamp 124 is closed. The first flow balance pump chamber 100 is empty and the second flow balance pump chamber 108 is filled with fresh dialysate solution (represented by the unshaded hatched fluid) previously prepared on the mixture part 34 of the cartridge 30. The dialyser 12 is filled on the blood side of the semi-permeable membrane with blood (represented by the fully shaded oppositely hatched fluid) and on the dialysate side of the semi-permeable membrane with dialysate solution which has drawn waste material out of the blood across the semi-permeable membrane (represented by the alternately shaded hatched fluid). At this point, the control system 500 operates the vacuum pumps 405 to 419 so as to close the valves 98, 102 and 120 of the first flow balance pump 100 and open valve 118 of the first flow balance pump and to close the valves 110, 112, 114 of the second flow balance pump 108 and to open valve 116 of the second flow balance pump 108. The controller then causes the empty flow balance pump 100 to be actuated to draw the spent dialysate from the dialyser 12 via the port 22 and valve 118 into the first flow balance pump chamber. The controller simultaneously causes the second flow balance pump to be actuated to push the clean dialysate solution from the second flow balance pump chamber via the valve 116 and port 104 and 20 into the dialyser.

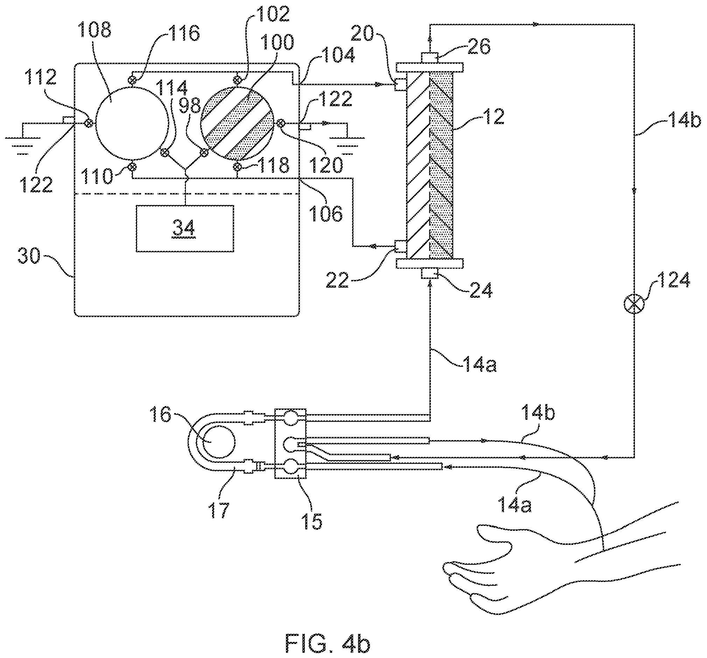

At this point in the method, as shown in Figure 4b, the first flow balance pump chamber 100 is filled with spent dialysate (as represented by the alternate shaded hatched fluid). The dialysate side of the semi-permeable membrane of the dialyser is filled with clean dialysate fluid (as represented by the unshaded hatched fluid) and the blood side of the dialyser is filled with blood (as represented by the fully shaded and oppositely hatched fluid).

The controller then operates the pneumatic pumps so as to close the valves 98, 102, 118 of the first flow balance pump 100 and the valves 110, 112 and 116 of the second flow balance pump 108 so that the spent dialysate outlet valve 120 of the first flow balance pump 100 and the clean dialysate inlet valve 114 of the second flow balance pump 108 are open. The controller actuates the flow balance pumps 100, 108 again so that the first flow balance pump expels the spent dialysate via the spent dialysate outlet valve 120 to the drain 122 and the second flow balance pump draws clean dialysate from the mixing part 34 of the cartridge 30 via the clean dialysate inlet valve 114 to fill the second flow balance pump 108 with clean dialysate fluid.

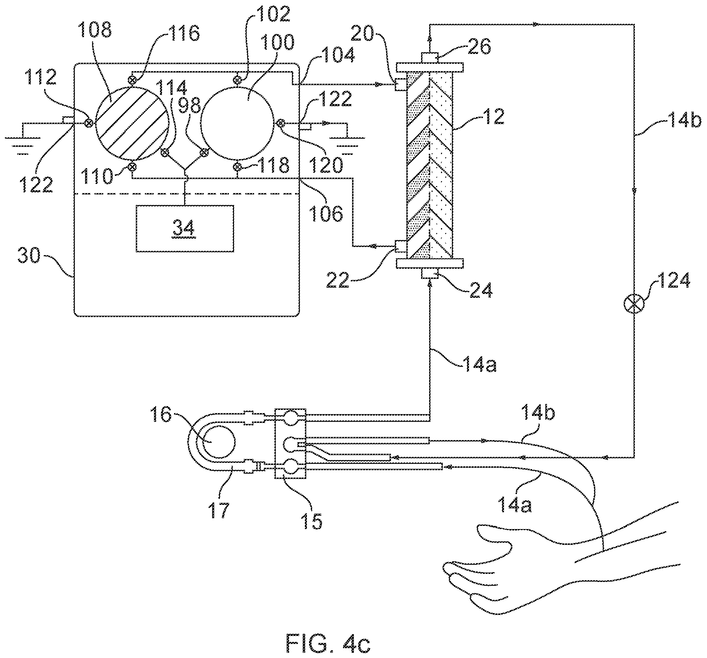

After that step, as represented in Figure 4c, the first flow balance pump chamber 100 is empty, the second flow balance pump chamber 108 is filled with clean dialysate solution (as illustrated by the unshaded hatched fluid), the dialysate side of the semi-permeable membrane of the dialyser 12 is filled with spent dialysate fluid (as illustrated by the alternately shaded hatched fluid) and the blood side of the semi-permeable membrane in the dialyser is filled with blood.

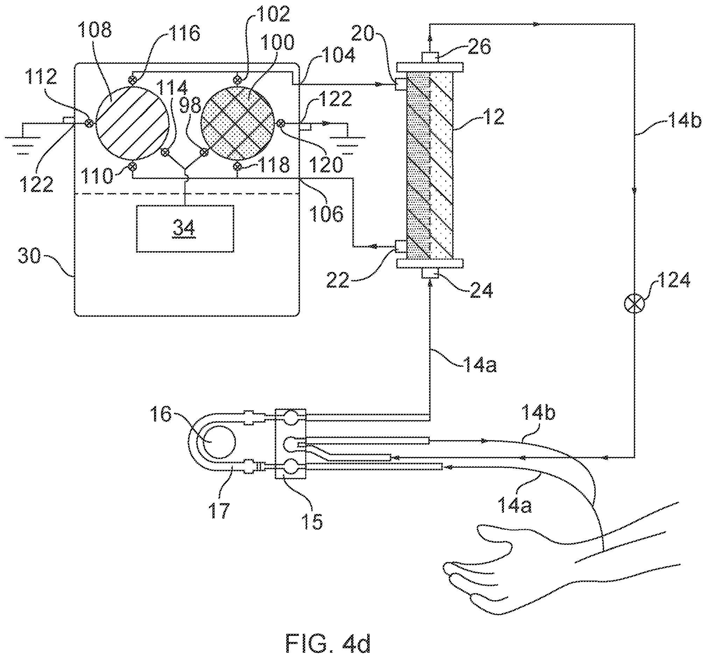

At this point, the controller closes the valves 98, 102 and 120 of the first flow balance pump, leaving valve 118 open and closes all of the valves 110, 112, 114, 116 of the second flow balance pump. The controller activates the peristaltic pump to draw blood from the patient via the venous access and the blood removal line 14a. The controller closes the venous clamp 124 so that the blood in the blood circuit becomes pressurised relative to the dialysate fluid in the dialysate circuit. Because of the pressure differential across the semi-permeable membrane in the dialyser, the blood plasma component of the blood in the dialyser 12 is forced across the semi-permeable membrane to mix with the spent dialysate solution on the dialysate side of the semi-permeable membrane and this, in turn, forces the spent dialysate blood component mixture into the first flow balance pump chamber 100. At this point, a proportion of the blood removed from the patient is on the dialysate side of the semi-permeable membrane in the first flow balance pump chamber. As such, the first flow balance pump chamber 100 is acting as a blood accumulator. This mixed dialysate solution/blood plasma is shown in Figure 4d as a fully shaded and cross-hatched fluid in the first flow balance pump chamber 100. Moreover, the action of pressurising the blood side of the dialyser forces blood plasma onto the dialysate side of the semi-permeable membrane in the dialyser as represented by the fully shaded hatched fluid in Figure 4d.

Then, the controller deactivates the peristaltic pump and acts to close the valves 98, 118 and 120 of the first flow balance pump, maintaining all of the valves of the second flow balance pump 108 closed and opening the venous clamp 124. The controller then causes the first flow balance pump 100 to be actuated, pushing the spent dialysate solution/blood plasma mixture back out of the first flow balance pump chamber (blood accumulator) and into the dialyser 12. The blood plasma component of the dialysate solution/blood plasma mixture will pass back across the semi-permeable membrane in the dialyser 12 and return via blood return line 14b to the patient. At the end of that step, the system returns to the condition shown in Figure 4a.

By means of this process, a single venous access can be used both for removal of blood from the patient and return of blood to the patient and by pushing blood across onto the dialysate side of the semi-permeable membrane, using the flow balance pump chambers as a blood accumulator, blood plasma is pushed across semi-permeable membrane which has a similar effect to haemodiafiltration.

A method of testing the system is disclosed and in such a case, the blood from a patient is replaced by a source of blood analogue fluid. The blood analogue fluid is a fluid which behaves in a similar fashion to blood. The blood analogue fluid preferably includes markers such as a dye to allow an observer to confirm that components of the blood analogue fluid are passed across the semi-permeable membrane into, and mixed together with, the spent dialysate solution then into the first flow balance pump chamber. Furthermore, the blood analogue fluid is preferably Dextran with marker molecules, such as fluorescein markers, which can be sensed using an appropriate sensor, such as a fluorometer. This enables the system to be tested to confirm that fluid is passed from the blood side of the semi-permeable membrane to the dialysate side of the semi-permeable membrane and that relevant molecules were removed from the blood analogue fluid when passed back across the semi-permeable membrane from the dialysate side to the blood side of the system.

## Claims

1. A dialysis system (10) comprising a dialysate fluid circuit and a blood circuit and a dialyser (12), the dialysate fluid circuit comprising a dialysate input pump having a dialysate input pump chamber, and a dialysate output pump having a dialysate output pump chamber,
the blood circuit comprising a blood pump (16) and a blood plasma accumulator chamber, a blood removal line (14a) upstream of the dialyser (12), a blood return line (14b) downstream of the dialyser (12) and a venous clamp (124) arranged on the blood return line (14b),
the dialyser (12) comprising a fluid chamber with a blood filter arranged in the fluid chamber to divide the system into a blood side (10b) and a dialysate side (10a), the dialyser (12) having a dialysate inlet (20) and a dialysate outlet (22) and a blood inlet (24) and a blood outlet (26),
the dialysate fluid circuit being arranged on the dialysate side (10a) of the system,
the blood plasma accumulator chamber being arranged on the dialysate side (10a) of the system (10), wherein the system (10) includes a control processor and **characterized in that** the control processor is configured to close the venous clamp (124) to pressurize blood in the blood circuit so that blood plasma is forced onto the dialysate side (10a) of the blood filter.

2. A dialysis system (10) according to claim 1 in which the dialysate output pump chamber comprises the blood plasma accumulator chamber.

3. A dialysis system (10) according to claim 1 or 2 in which the dialysate input pump chamber has a dialysate inlet, with a dialysate inlet valve, in fluid communication with a source of dialysate (76) and a dialysate outlet, with a dialysate outlet valve, in fluid communication with the dialysate inlet (20) of the dialyser (12).

4. A dialysis system (10) according to any of claims 1 to 3 in which the dialysate output pump chamber has a spent dialysate inlet, with a spent dialysate inlet valve, in fluid communication with the dialysate outlet of the dialyser (12) and a spent dialysate outlet, with a spent dialysate outlet valve, in fluid communication with a drain (122).

5. A dialysis system (10) according to claim 1, in which the dialysate fluid circuit comprises a first pump (100) having a first pump chamber with a fresh dialysate inlet, with a fresh dialysate inlet valve (98), in fluid communication with a source of dialysate (76), a dialyser fluid line, with a dialyser fluid valve (102), in fluid communication with dialyser (12) and a drain line with a drain valve (120), in fluid communication with a drain (122) and a second pump (108) having a second pump chamber with a fresh dialysate inlet, with a fresh dialysate inlet valve (114), in fluid communication with a source of dialysate (76), a dialyser fluid line, with a dialyser fluid valve (116), in fluid communication with a dialyser (12) and a drain line with a drain valve (112) in fluid communication with a drain (122), wherein the a control processor controls the valves whereby the first pump (100) can be configured to operate as the dialysate input pump by closing off the drain valve (120) and the second pump (108) is then configured to operate as the dialysate output pump by closing off the fresh dialysate inlet valve (114) and vice versa.

6. A dialysis system (10) according to claim 5, in which the pump chamber of either pump (100, 108) can be configured to operate as the blood plasma accumulator chamber by closing off the respective drain valve (120, 112) and the respective fresh dialysate inlet valve (98, 114) thereof.

7. A dialysis system (10) according to any preceding claim, in which the dialysate fluid circuit comprises a body defining the pump chambers closed by a membrane actuable to effect pumping.

8. A dialysis system (10) according to claim 7 in which the body and membrane forms a cartridge (30) for use with a machine including actuators to actuate the membrane to effect pumping.

9. A dialysis system (10) according to claim 8 and claim 3 and/or claim 4 in which the valves (98, 102, 120, 112, 114, 116) are defined on the body and the valves (98, 102, 120, 112, 114, 116) are opened and closed by actuation of the membrane.

10. A dialysis system according to claim 8 or 9 in which the cartridge (30) also includes at least one mixing chamber (34) to effect mixing of dialysate constituents into a mixed dialysate and the mixing chamber (34) is in fluid communication with the dialysate input pump.

11. A dialysis system (10) according to any preceding claim, wherein the blood removal line (14a) and the blood inlet line (14b) converge to a single venous access line in blood communication with a patient.

12. A method of testing a dialysis system (10) comprising the steps of a) providing a dialysis system (10) according to claim 1, b) providing a source (76) of dialysate fluid, c) providing a source of blood analogue fluid, d) supplying the dialysate fluid to the dialysate circuit and supplying the blood analogue fluid to the blood circuit, e) operating the dialysate outlet pump to draw blood analogue fluid and/or operating the blood pump (16) to push blood analogue fluid across the blood filter onto the dialysate side (10a) and into the dialysate outlet pump chamber, f) deactivating the blood pump (16) and operating the dialysate outlet pump to push blood analogue fluid back across the blood filter onto the blood side (10b) of the system (10).

13. The method of testing a dialysis system (10) according to claim 12 in which step e) comprises a first pre-step e' in which the blood pump (16) is deactivated, the venous clamp (124) is closed to prevent flow of blood analogue fluid from the dialyser (12) to the blood return line (14b), the dialysate input pump chamber is filled with dialysate, the dialysate outlet valve of the dialysate input pump chamber is open, the dialysate output pump chamber is empty, the spent dialysate inlet valve is open and the spent dialysate outlet valve is closed, the first pre-step comprising actuating the dialysate input pump to push fresh dialysate from the dialysate input pump chamber to the dialyser (12) and simultaneously actuating the dialysate output pump to draw spent dialysate from the dialyser (12) into the dialysate output pump chamber, a second pre-step e", in which the blood pump (16) is deactivated, the venous clamp (124) is closed, the dialysate input pump chamber is empty and the dialysate output pump chamber is filled with spent dialysate, pre-step e" comprising opening the dialysate inlet valve of the dialysate input pump, closing the dialysate outlet valve of the dialysate input pump, closing the spent dialysate inlet valve of the dialysate output pump, opening the spent dialysate output valve of the dialysate output pump, then activating dialysate input pump to flow fresh dialysate from the dialysate source (76) into the dialysate input pump chamber and simultaneously activating the dialysate output pump to push spent dialysate to drain (122).

14. A dialysis system (10) according to claim 1, wherein:
the dialysate fluid circuit comprises first and second pump chambers each having a plurality of respective valves configurable to enable or prevent fluid communication with both the dialysate side (10a) of the dialyser (12) and a drain (122),
each of the first and second pump chambers are configurable, based on the configurations of the valves, to comprise the blood plasma accumulation chamber, and
the dialysis system (10) being **characterized in that** the blood removal line (14a) and blood return line (14b) converge to a single venous access line in blood communication with a patient and a venous clamp (124) arranged on the blood return line (14b).

## Patentansprüche

1. Dialysesystem (10) aufweisend einen Dialysatfluid-Kreislauf und einen Blutkreislauf und einen Dialysator (12), wobei
der Dialysatfluid-Kreislauf eine Dialysat-Einspeisepumpe mit einer Dialysat-Einspeisepumpenkammer und eine Dialysat-Ausspeisepumpe mit einer Dialysat-Ausspeisepumpenkammer aufweist,
der Blutkreislauf eine Blutpumpe (16) und eine Blutplasma-Sammelkammer, eine Blutentnahmeleitung (14a) stromauf des Dialysators (12), eine Blutrücklaufleitung (14b) stromab des Dialysators (12) und eine an der Blutrücklaufleitung (14b) angeordnete Venenklammer (124) aufweist,
der Dialysator (12) eine Fluidkammer mit einem in der Fluidkammer angeordneten Blutfilter aufweist, um das System in eine Blutseite (10b) und eine Dialysatseite (10a) zu unterteilen, der Dialysator (12) einen Dialysat-Einlass (20) und einen Dialysat-Auslass (22) und einen Bluteinlass (24) und einen Blutauslass (26) hat,
der Dialysatfluid-Kreislauf an der Dialysatseite (10a) des Systems angeordnet ist,
die Blutplasma-Sammelkammer an der Dialysatseite (10a) des Systems (10) angeordnet ist, wobei das System (10) einen Steuerprozessor aufweist und **dadurch gekennzeichnet ist, dass** der Steuerprozessor konfiguriert ist, die Venenklammer (124) zu schließen, um Druck auf das Blut in dem Blutkreislauf auszuüben, so dass Blutplasma auf die Dialysatseite (10a) des Blutfilters gezwungen wird.

2. Dialysesystem (10) nach Anspruch 1, in welchem die Dialysat-Ausspeisepumpenkammer die Blutplasma-Sammelkammer aufweist.

3. Dialysesystem (10) nach Anspruch 1 oder 2, in welchem die Dialysat-Einspeisepumpenkammer einen Dialysat-Einlass mit einem Dialysat-Einlassventil in Fluidverbindung mit einer Dialysatquelle (76) und einen Dialysat-Auslass mit einem Dialysat-Auslassventil in Fluidverbindung mit dem Dialysat-Einlass (20) des Dialysators (12) hat.

4. Dialysesystem (10) nach einem der Ansprüche 1 bis 3, in welchem die Dialysat-Ausspeisepumpenkammer einen Altdialysat-Einlass mit einem Altdialysat-Einlassventil in Fluidverbindung mit dem Dialysat-Auslass des Dialysators (12) und einen Altdialysat-Auslass mit einem Altdialysat-Auslassventil in Fluidverbindung mit einem Ablauf (122) hat.

5. Dialysesystem (10) nach Anspruch 1, in welchem der Dialysatfluid-Kreislauf aufweist:
eine erste Pumpe (100), die eine erste Pumpenkammer mit einem Frischdialysat-Einlass mit einem Frischdialysat-Einlassventil (98) in Fluidverbindung mit einer Dialysatquelle (76), eine Dialysatorfluidleitung mit einem Dialysatorfluidventil (102) in Fluidverbindung mit dem Dialysator (12) und eine Ablaufleitung mit einem Ablaufventil (120) in Fluidverbindung mit einem Ablauf (122) hat, und
eine zweite Pumpe (108), die eine zweite Pumpenkammer mit einem Frischdialysat-Einlass mit einem Frischdialysat-Einlassventil (114) in Fluidverbindung mit einer Dialysatquelle (76), eine Dialysatorfluidleitung mit einem Dialysatorfluidventil (116) in Fluidverbindung mit dem Dialysator (12) und eine Ablaufleitung mit einem Ablaufventil (112) in Fluidverbindung mit einem Ablauf (122) hat,
wobei ein Steuerprozessor die Ventile steuert, wodurch die erste Pumpe (100) konfiguriert sein kann, durch Schließen des Ablaufventils (120) als die Dialysat-Einspeisepumpe zu arbeiten, und die zweite Pumpe (108) dann konfiguriert ist, durch Schließen des Frischdialysat-Einlassventils (114) als die Dialysat-Ausspeisepumpe zu arbeiten, und vice versa.

6. Dialysesystem (10) nach Anspruch 5, in welchem die Pumpenkammer jeder Pumpe (100, 108) konfiguriert sein kann, durch Schließen ihres jeweiligen Ablaufventils (120, 112) und ihres jeweiligen Frischdialysat-Einlassventils (98, 114) als die Blutplasma-Sammelkammer zu arbeiten.

7. Dialysesystem (10) nach einem der vorstehenden Ansprüche, in welchem der Dialysatfluid-Kreislauf einen Körper aufweist, der die Pumpenkammern definiert, die mittels einer betätigbaren Membran geschlossen werden, um Pumpen durchzuführen.

8. Dialysesystem (10) nach Anspruch 7, in welchem der Körper und die Membran eine Kartusche (30) bilden, die mit einer Maschine verwendbar ist, die Aktuatoren aufweist, um die Membran zu betätigen, um Pumpen durchzuführen.

9. Dialysesystem (10) nach Anspruch 8 und Anspruch 3 und/oder Anspruch 4, in welchem die Ventile (98, 102, 120, 112, 114, 116) an dem Körper definiert sind und die Ventile (98, 102, 120, 112, 114, 116) durch Betätigung der Membran geöffnet und geschlossen werden.

10. Dialysesystem nach Anspruch 8 oder 9, in welchem die Kartusche (30) außerdem mindestens eine Mischkammer (34) aufweist, um Mischen von Dialysatbestandteilen zu einem gemischten Dialysat durchzuführen, und die Mischkammer (34) in Fluidverbindung mit der Dialysat-Einspeisepumpe ist.

11. Dialysesystem (10) nach einem der vorstehenden Ansprüche, wobei die Blutentnahmeleitung (14a) und die Blutrücklaufleitung (14b) zu einer einzigen Venenzugangsleitung in Blutverbindung mit dem Patienten zusammenlaufen.

12. Verfahren zum Testen eines Dialysesystems (10) aufweisend die Schritte
a) Bereitstellen eines Dialysesystems (10) nach Anspruch 1,
b) Bereitstellen einer Dialysatfluidquelle (76),
c) Bereitstellen einer Quelle von blutanalogem Fluid,
d) Zuführen des Dialysatfluids zu dem Dialysatkreislauf und Zuführen des blutanalogen Fluids zu dem Blutkreislauf,
e) Betreiben der Dialysat-Ausspeisepumpe, um blutanaloges Fluid zu saugen, und/oder Betreiben der Blutpumpe (16), um blutanaloges Fluid über das Blutfilter auf die Dialysatseite (10a) und in die Dialysat-Ausspeisepumpenkammer zu drücken,
f) Deaktivieren der Blutpumpe (16) und Betreiben der Dialysat-Ausspeisepumpe, um blutanaloges Fluid über das Blutfilter zurück auf die Blutseite (10b) des Systems (10) zu drücken.

13. Verfahren zum Testen eines Dialysesystems (10) nach Anspruch 12, in welchem Schritt e) aufweist:
einen ersten Vorschritt e', in welchem
die Blutpumpe (16) deaktiviert ist,
die Venenklammer (124) geschlossen ist, um ein Fließen von blutanalogem Fluid aus dem Dialysator (12) zu der Blutrücklaufleitung (14b) zu verhindern,
die Dialysat-Einspeisepumpenkammer mit Dialysat gefüllt ist,
das Dialysat-Auslassventil der Dialysat-Einspeisepumpenkammer offen ist,
die Dialysat-Ausspeisepumpenkammer leer ist,
das Altdialysat-Einlassventil offen ist und das Altdialysat-Auslassventil geschlossen ist,
wobei der erste Vorschritt aufweist:
Betätigen der Dialysat-Einspeisepumpe, um Frischdialysat aus der Dialysat-Einspeisepumpenkammer zu dem Dialysator (12) zu drücken und gleichzeitiges Betätigen der Dialysat-Ausspeisepumpenkammer, um Altdialysat aus dem Dialysator (12) in die Dialysat-Ausspeisepumpenkammer zu drücken,
einen zweiten Vorschritt e", in welchem
die Blutpumpe (16) deaktiviert ist,
die Venenklammer (124) geschlossen ist,
die Dialysat-Einspeisepumpenkammer leer ist und die Dialysat-Ausspeisepumpenkammer mit Altdialysat gefüllt ist,
wobei der Vorschritt e" aufweist:
Öffnen des Dialysat-Einlassventils der Dialysat-Einlasspumpe,
Schließen des Dialysat-Auslassventils der Dialysat-Einlasspumpe,
Schließen des Altdialysat-Einlassventils der Dialysat-Ausspeisepumpe,
Öffnen des Altdialysat-Auslassventils der Dialysat-Ausspeisepumpe,
dann Aktivieren der Dialysat-Einspeisepumpe, um Frischdialysat aus der Dialysatquelle (76) in die Dialysat-Einspeisepumpenkammer fließen zu lassen und gleichzeitiges Aktivieren der Dialysat-Ausspeisepumpe, um Altdialysat zum Ablauf (122) zu drücken.

14. Dialysesystem (10) nach Anspruch 1, wobei:
der Dialysatfluid-Kreislauf erste und zweite Pumpenkammern aufweist, die jeweils mehrere jeweilige Ventile haben, die konfigurierbar sind, eine Fluidverbindung mit sowohl der Dialysatseite (10a) des Dialysators (12) als auch einem Ablauf (122) zu ermöglichen oder zu verhindern,
wobei jede der ersten und zweiten Pumpenkammern auf Basis der Konfigurationen der Ventile konfigurierbar ist, um die Blutplasma-Sammelkammer aufzuweisen, und
das Dialysesystem (10) **dadurch gekennzeichnet ist, dass** die Blutentnahmeleitung (14a) und die Blutrücklaufleitung (14b) zu einer einzigen Venenzugangsleitung in Blutverbindung mit einem Patienten und einer an der Blutrücklaufleitung (14b) angeordneten Venenklammer (124) zusammenlaufen.

## Revendications

1. Système de dialyse (10) comprenant un circuit de fluide de dialysat et un circuit de sang et un dialyseur (12), le circuit de fluide de dialysat comprenant une pompe d'entrée de dialysat ayant une chambre de pompe d'entrée de dialysat, et une pompe de sortie de dialysat ayant une chambre de pompe de sortie de dialysat,
le circuit de sang comprenant une pompe de sang (16) et une chambre d'accumulation de plasma sanguin, une ligne de prélèvement de sang (14a) en amont du dialyseur (12), une ligne de retour de sang (14b) en aval du dialyseur (12) et une pince veineuse (124) agencée sur la ligne de retour de sang (14b),
le dialyseur (12) comprenant une chambre de fluide avec un filtre sanguin agencé dans la chambre de fluide pour diviser le système en un côté de sang (10b) et en un côté de dialysat (10a), le dialyseur (12) ayant une entrée de dialysat (20) et une sortie de dialysat (22) et une entrée de sang (24) et une sortie de sang (26),
le circuit de fluide de dialysat étant agencé du côté de dialysat (10a) du système,
la chambre d'accumulation de plasma sanguin étant agencée du côté de dialysat (10a) du système (10), dans lequel le système (10) comprend un processeur de commande et **caractérisé en ce que** le processeur de commande est configuré pour fermer la pince veineuse (124) pour mettre le sang sous pression dans le circuit de sang de sorte que le plasma sanguin est forcé du côté de dialysat (10a) du filtre sanguin.

2. Système de dialyse (10) selon la revendication 1, dans lequel la chambre de pompe de sortie de dialysat comprend la chambre d'accumulation de plasma sanguin.

3. Système de dialyse (10) selon la revendication 1 ou 2, dans lequel la chambre de pompe d'entrée de dialysat a une entrée de dialysat, avec une valve d'entrée de dialysat, en communication de fluide avec une source de dialysat (76) et une sortie de dialysat, avec une valve de sortie de dialysat, en communication de fluide avec l'entrée de dialysat (20) du dialyseur (12).

4. Système de dialyse (10) selon l'une quelconque des revendications 1 à 3, dans lequel la chambre de pompe de sortie de dialysat a une entrée de dialysat usagé, avec une valve d'entrée de dialysat usagé, en communication de fluide avec la sortie de dialysat du dialyseur (12) et une sortie de dialysat usagé, avec une valve de sortie de dialysat usagé, en communication de fluide avec un drain (122).

5. Système de dialyse (10) selon la revendication 1, dans lequel le circuit de fluide de dialysat comprend une première pompe (100) ayant une première chambre de pompe avec une entrée de dialysat frais, avec une valve d'entrée de dialysat frais (98) en communication de fluide avec une source de dialysat (76), une ligne de fluide de dialyseur, avec une valve de fluide de dialyseur (102) en communication de fluide avec le dialyseur (12) et une ligne de drain avec une valve de drain (120) en communication de fluide avec un drain (122) et une seconde pompe (108) ayant une seconde chambre de pompe avec une entrée de dialysat frais, avec une valve d'entrée de dialysat frais (114) en communication de fluide avec une source de dialysat (76), une ligne de fluide de dialyseur avec une valve de fluide de dialyseur (116) en communication de fluide avec un dialyseur (12) et une ligne de drain avec une valve de drain (112) en communication de fluide avec un drain (122), dans lequel le un processeur de commande commande les valves, moyennant quoi la première pompe (100) peut être configurée pour fonctionner en tant que pompe d'entrée de dialysat en fermant la valve de drain (120) et la seconde pompe (108) est ensuite configurée pour fonctionner en tant que pompe de sortie de dialysat en fermant la valve d'entrée de dialysat frais (114) et vice versa.

6. Système de dialyse (10) selon la revendication 5, dans lequel la chambre de pompe de chaque pompe (100, 108) peut être configurée pour fonctionner en tant que chambre d'accumulation de plasma sanguin en fermant la valve de drain (120, 112) respective et sa valve d'entrée de dialysat frais (98, 114) respective.

7. Système de dialyse (10) selon l'une quelconque des revendications précédentes, dans lequel le circuit de fluide de dialysat comprend un corps définissant les chambres de pompe fermées par une membrane pouvant être actionnée pour effectuer le pompage.

8. Système de dialyse (10) selon la revendication 7, dans lequel le corps et la membrane forment une cartouche (30) destinée à être utilisée avec une machine comprenant des actionneurs afin d'actionner la membrane pour effectuer le pompage.

9. Système de dialyse (10) selon la revendication 8 et la revendication 3 et/ou la revendication 4, dans lequel les valves (98, 102, 120, 112, 114, 116) sont définies sur le corps et les valves (98, 102, 120, 112, 114, 116) sont ouvertes et fermées par l'actionnement de la membrane.

10. Système de dialyse (10) selon la revendication 8 ou 9, dans lequel la cartouche (30) comprend également au moins une chambre de mélange (34) pour effectuer le mélange des constituants de dialysat dans un dialysat mélangé et la chambre de mélange (34) est en communication de fluide avec la pompe d'entrée de dialysat.

11. Système de dialyse (10) selon l'une quelconque des revendications précédentes, dans lequel la ligne de prélèvement de sang (14a) et la ligne d'entrée de sang (14b) convergent vers une ligne d'accès veineux en communication de fluide avec un patient.

12. Procédé pour tester un système de dialyse (10) comprenant les étapes consistant à a) prévoir un système de dialyse (10) selon la revendication 1, b) prévoir une source (76) du fluide de dialysat, c) prévoir une source de fluide analogue du sang, d) amener le fluide de dialysat au circuit de dialysat et amener le fluide analogue du sang au circuit de sang, e) actionner la pompe de sortie de dialysat pour aspirer le fluide analogue du sang et/ou actionner la pompe de sang (16) afin de pousser le fluide analogue du sang sur le filtre sanguin du côté de dialysat (10a) et dans la chambre de pompe de sortie de dialysat, f) désactiver la pompe de sang (16) et actionner la pompe de sortie de dialysat afin de repousser le fluide analogue du sang sur le filtre sanguin du côté de sang (10b) du système (10).

13. Procédé pour tester un système de dialyse (10) selon la revendication 12, dans lequel l'étape e) comprend une première pré-étape e' dans laquelle la pompe de sang (16) est désactivée, la pince veineuse (124) est fermée pour empêcher l'écoulement du fluide analogue du sang du dialyseur (12) à la ligne de retour de sang (14b), la chambre de pompe d'entrée de dialysat est remplie avec le dialysat, la valve de sortie de dialysat de la chambre de pompe d'entrée de dialysat est ouverte, la chambre de pompe de sortie de dialysat est vide, la valve d'entrée de dialysat usagé est ouverte et la valve de sortie de dialysat usagé est fermée, la première pré-étape comprenant les étapes consistant à actionner la pompe d'entrée de dialysat pour pousser le dialysat frais de la chambre de pompe d'entrée de dialysat au dialyseur (12) et actionner simultanément la pompe de sortie de dialysat pour aspirer le dialysat usagé du dialyseur (12) dans la chambre de pompe de sortie de dialysat, une seconde pré-étape e", dans laquelle la pompe de sang (16) est désactivée, la pince veineuse (124) est fermée, la chambre de pompe d'entrée de dialysat est vide et la chambre de pompe de sortie de dialysat est remplie avec le dialysat usagé, la pré-étape e" comprenant les étapes consistant à ouvrir la valve d'entrée de dialysat de la pompe d'entrée de dialysat, fermer la valve de sortie de dialysat de la pompe d'entrée de dialysat, fermer la valve d'entrée de dialysat usagé de la pompe de sortie de dialysat, ouvrir la valve de sortie de dialysat usagé de la pompe de sortie de dialysat, activer ensuite la pompe d'entrée de dialysat pour laisser s'écouler le dialysat frais de la source de dialysat (76) dans la chambre de pompe d'entrée de dialysat et activer simultanément la pompe de sortie de dialysat pour pousser le dialysat usagé dans le drain (122).

14. Système de dialyse (10) selon la revendication 1, dans lequel :
le circuit de fluide de dialysat comprend des première et seconde chambres de pompe ayant chacune une pluralité de valves respectives pouvant être configurées pour permettre ou empêcher la communication de fluide à la fois avec le côté de dialysat (10a) du dialyseur (12) et un drain (122),
chacune des première et seconde chambres de pompe peut être configurée, sur la base des configurations des valves, pour comprendre la chambre d'accumulation de plasma sanguin, et
le système de dialyse (10) étant **caractérisé en ce que** la ligne de prélèvement de sang (14a) et la ligne de retour de sang (14b) convergent vers une seule ligne d'accès veineux en communication de fluide avec un patient et une pince veineuse (124) agencée dans la ligne de retour de sang (14b).
